(19) Europäisches Patentamt
European Patent Office
Office européen des brevets

(11) Veröffentlichungsnummer: **0 544 074 A1**

(12) # EUROPÄISCHE PATENTANMELDUNG

(21) Anmeldenummer: **92116506.4**

(22) Anmeldetag: **26.09.92**

(51) Int. Cl.5: **C07C 37/14**, C07C 39/06, C07C 39/17

(30) Priorität: **28.11.91 DE 4139056**

(43) Veröffentlichungstag der Anmeldung: **02.06.93 Patentblatt 93/22**

(84) Benannte Vertragsstaaten: **CH DE FR GB IT LI NL**

(71) Anmelder: **HÜLS AKTIENGESELLSCHAFT**
**Patentabteilung / PB 15 - Postfach 13 20**
**W-4370 Marl 1(DE)**

(72) Erfinder: **Küpper, Friedrich-Wilhelm, Dr.**
**Oderbruchstrasse 27**
**W-4370 Marl(DE)**
Erfinder: **Müller, Wolfgang, Dr.**
**Kaspar-Grove-Strasse 32**
**W-4370 Marl(DE)**

(54) **Verfahren zur Herstellung von ortho-substituierten Alkylphenolen und Katalysator hierfür.**

(57) Die Erfindung betrifft ein Verfahren zur Herstellung von ortho-substituierten Alkylphenolen mit Katalysatoren erhöhter Aktivität, durch Umsetzung von Phenol oder 2-Alkyl-Phenolen mit 2-Alkyl- bzw. 2-Aryl-1-alkenen in der Flüssigphase, insbesondere in Gegenwart geeigneter inerter Verdünnungsmittel und/oder eines Überschusses an umzusetzendem Alken, bei Temperaturen von 0 ° bis 100 °C und Drücken von 0,1 bis 20 bar, welches dadurch gekennzeichnet ist, daß man die Umsetzung in Gegenwart modifizierter aluminiumhaltiger Katalysatoren durchführt, die durch Zusatz kleiner Mengen als Cokatalysatoren wirkender Polyhalogenphenole mit mindestens drei und vorzugsweise fünf gleichen oder verschiedenen Halogensubstituenten (aus der Gruppe der Elemente Fluor, Chlor und Brom) zu an sich bekannten Aluminiumtrisphenolatkatalysatoren erhalten werden, wobei die Katalysatorformierung in Gegenwart der Cokatalysatoren vorgenommen wird oder die Cokatalysatoren zum bereits formierten Katalysator vor Beginn der Alkylierung in solchen Mengen zugesetzt werden, daß das molare Verhältnis zwischen Cokatalysator und Aluminiumverbindung 0,5 : 1 bis 8 : 1, vorzugsweise 1 : 1 bis 6 : 1, und besonders bevorzugt 2 : 1 bis 3 : 1, beträgt.

Die Erfindung betrifft außerdem den Katalysator zur Herstellung der ortho-alkylierten Phenole, gebildet aus dem zu alkylierenden (2-Alkyl)Phenol, einer aluminiumorganischen Verbindung und einem Polyhalogenphenol als Cokatalysator.

EP 0 544 074 A1

Die Erfindung betrifft ein Verfahren zur Herstellung von Alkylphenolen mit neuen aluminiumhaltigen homogenen Katalysatoren, deren Aktivität durch Zusatz von Polyhalogenphenolen als Cokatalysatoren gesteigert wird, so daß die mit diesen Katalysatoren gegebenenfalls auch in Gegenwart von gesättigten aliphatischen und/oder cycloaliphatischen Kohlenwasserstoffen und/oder überschüssigem flüssigem bzw. gelöstem Alken mit letzterem durchzuführenden Alkylierungen von Phenol, vor allem aber von 2-Alkylphenolen, bei niedrigeren Temperaturen und niedrigeren Drücken rascher verlaufen als mit herkömmlichen Aluminiumphenolatkatalysatoren. Dadurch kann die Herstellung der vorzugsweise gewünschten 2,6-Dialkylphenole bei milderen Bedingungen und/oder mit den aus Gründen der Wirtschaftlichkeit angestrebten besseren Raum-Zeit-Ausbeuten erfolgen. Die Erfindung betrifft auch den Katalysator selbst.

Es ist bekannt, daß ortho-substituierte bzw. di-ortho-substituierte Alkylphenole durch Anlagerung von Alkenen an Phenol, 2-Alkylphenole und andere Hydroxyaromaten in Gegenwart von Aluminiumphenolaten gewonnen werden können (vgl. Ullmann, "Enzyklopädie der Technischen Chemie", Band 18 (1979) S. 200 ff.). Üblicherweise wird die Alkylierung von Phenol in Gegenwart von Aluminiumphenolat bei mindestens 100 °C und gegebenenfalls unter erhöhtem Druck durchgeführt. Der jeweils zu wählende optimale Temperaturbereich hängt von der Substitution der C = C-Bindung im anzulagernden Alken ab. Stets wird eine möglichst stark verzweigte Alkylgruppe gebildet (vgl. loc. cit.). Isobuten und andere zur Bildung tertiärer Alkylsubstituenten führende Alkene sind besonders reaktiv. Daher wird beispielsweise die Anlagerung von Isobuten an Phenol bzw. 2-Tert.butylphenol meist bei 110 ° bis 120 °C unter erhöhtem Druck durchgeführt, der bis zu 25 bar betragen soll, während für Alkene wie Propen, Cyclopenten oder Cyclohexen Reaktionstemperaturen von mehr als 180 °C erforderlich sind.

Der Katalysator wird durch Lösen von 1 bis 3 Gew.-% Aluminium im zu alkylierenden Phenol gewonnen (vgl. US-PS 2 831 898, DE-PSS 944 014, 1 044 825, J.Org. Chem. 22 (1957) S. 642; Ang. Chem. 69 (1957) S. 699). Er kann aber auch durch Umsetzung von Aluminiumalkoholaten oder von aluminiumorganischen Verbindungen mit dem Phenol sowie auf weiteren Wegen hergestellt werden. Die Phenolate des Aluminiums nehmen unter der Vielzahl der in der Patentliteratur genannten Metallphenolate hinsichtlich der erreichbaren Selektivität und Aktivität eine Sonderstellung ein. Dennoch müssen auch Aluminiumphenolatkatalysatoren in relativ großen Mengen eingesetzt werden, um zu wirtschaftlich interessanten Raum-Zeit-Ausbeuten zu gelangen.

Nachteilig bei den älteren Verfahren des Standes der Technik ist außerdem, daß die erforderlichen größeren Mengen der nur mäßig aktiven Aluminium-tris-(phenolat)-Katalysatoren vor der destillativen Trennung der Reaktionsprodukte desaktiviert werden müssen. Die Beseitigung der dabei anfallenden, Aluminiumverbindungen und (Alkyl-)Phenole enthaltenden Abwässer ist aus Gründen des Umweltschutzes unbedingt erforderlich, aber nicht unproblematisch. Dies deutet schon die Zahl der Patentanmeldungen an, die sich bis in die jüngste Zeit mit der Desaktivierung der Katalysatoren sowie mit teilweise recht aufwendigen Lösungen bei der Entsorgung des Abwassers bzw. mit der Verringerung der anfallenden Mengen befassen (vgl. z. B. US-PS 3 200 157, DE-PS 1 809 555, DE-OS 20 039 062, US-PS 3 939 215, DE-PS 26 02 149, BE-PS 842 691, US-PS 3 652 685, US-PS 3 970 708). Die mit der Abwasserbeseitigung verbundenen Probleme waren bisher auch durch die Heterogenisierung bekannter Katalysatoren nicht zu lösen (vgl. EP-PS 0 206 085), da die katalytisch wirksamen Aluminiumphenolatkatalysatoren mit den Reaktionsprodukten in nicht zu vernachlässigendem Umfang ausgetragen werden.

Nur in wenigen Patenten werden Maßnahmen beschrieben, durch die die erwähnte mäßige Aktivität der Aluminium-tris-(phenolat)-katalysatoren angeblich gesteigert werden konnte. So soll der Zusatz von Metallhalogeniden, vorzugsweise von Chloriden der Alkalien, der Erdalkalien und des Aluminiums (vgl. DE-PS 1 044 825), aber auch von Alkylhalogeniden (vgl. US-PSS 3 426 082, 3 200 157), von Alkaliphenolaten (vgl. FR-PS 1 331 450, JA-OS 6 100 0036 (s. CA-Referat 104 (1986) 186 127)) sowie von stickstoff- bzw. phosphorhaltigen Cokatalysatoren (vgl. JA-OS 60 218 346 (s. CA-Referat 104 (1986) 88273) von Vorteil sein. Die den Beispielen der genannten Patentschriften zu entnehmenden Effekte sind wenig überzeugend, zumal unverändert hohe Reaktionstemperaturen anzuwenden sind, daher gegebenenfalls unter höherem Druck gearbeitet werden muß und unter diesen Bedingungen in Anwesenheit der halogenhaltigen Zusätze die häufig unerwünschten para-Alkylphenolisomeren verstärkt gebildet werden. Ein Hinweis auf die mäßige Wirkung der erwähnten Zusätze könnte auch sein, daß über deren Verwendung bei der Produktion von Alkylphenolen nie etwas bekannt wurde.

Lediglich für den Fall der Katalysatorformierung aus aluminiumorganischen Verbindungen, vorzugsweise aus Aluminiumalkylen, und 2-Tert.butylphenol wurde ein Katalysator beschrieben, der bereits erheblich unterhalb von 100 °C katalytisch aktiv ist (vgl. US-PS 3 355 504). Mit diesem System gelingt die Isobutenanlagerung von 2-Tert.butylphenol, das an Stelle von Phenol einzusetzen ist, auch noch bei 10 °C. Die Alkylierung führt trotz der niedrigen Reaktionstemperaturen rasch zum gewünschten 2,6-Ditert.butylphenol. Dabei fällt diese Verbindung bei Verwendung vergleichbarer Katalysatormengen (ca. 1-3 mol-%, bezogen auf eingesetztes 2-Tert.butylphenol) mit etwas geringeren Anteilen unerwünschter Beimengungen (wie 2,4-Ditert.butylphenol oder 2,4,6-Tritert.butylphenol) an. Wie alle anderen bekannten Phenolalkylierungskatalysatoren ist auch dieses spezielle System vor Beginn der Aufarbeitung zu desaktivieren, um De- und Umalkylierungen während der destillativen Trennung der Reaktionsprodukte zu vermeiden. Die Selektivität der Bildung von 2,6-Ditert.butylphenol aus Phenol bzw. 2-Tert.butylphenol läßt sich durch verschiedene Maßnahmen (vgl. EP-OS 0 347 709, EP-OS 0 347 710 und insbesondere DE-PS 39 41 472) erheblich gegenüber dem erwähnten Stand der Technik verbessern, so daß auf Grund der verbesserten Ausbeuten auch die Wirtschaftlichkeit der 2,6-Ditert.butylphenolherstellung günstig beeinflußt wird. In allen Fällen wird der Einsatz der bekannten Katalysatoren empfohlen.

Die Verfahren des Standes der Technik setzen überwiegend nur mäßig aktive Katalysatoren ein, deren Verwendung mit den geschilderten Nachteilen bei den notwendigen hohen Temperaturen, wie beträchtlichen Anteilen unerwünschter Nebenprodukte sowie mit der problematischen Beseitigung erheblicher Mengen der desaktivierten Kontakte verbunden ist.

Es bestand daher die Aufgabe der Erfindung darin, Katalysatoren mit deutlich verbesserter Aktivität für die Herstellung von ortho-substituierten Alkylphenolen zu entwickeln, die gegebenenfalls in wesentlich geringerer Menge als bisher eingesetzt werden können und mit denen die gewünschten Umsetzungen auch bei niedrigen Temperaturen genügend schnell verlaufen.

Die Lösung dieser Aufgabe gelingt in einer keinen zusätzlichen technischen Aufwand erfordernden Weise, indem die Herstellung der aluminiumhaltigen Katalysatoren für das Verfahren zur Herstellung von ortho-alkylierten Phenolen in Gegenwart zugesetzter kleiner Mengen ausgewählter Polyhalogenphenole vorgenommen wird, ehe die gegebenenfalls in Gegenwart aliphatischer und/oder cycloaliphatischer Kohlenwasserstoffe und/ oder überschüssigem flüssigem bzw. gelöstem Alken durchzuführende Alkylierung von Phenol bzw. von Alkylphenolen, insbesondere 2-Alkylphenolen, die auch in 3- und/oder 4-Position substituiert sein können, erfolgt.
Besonders geeignet ist das Verfahren zur Herstellung von 2,6-Dialkylphenolen, insbesondere von 2,6-Ditert.butylphenol oder von neuen Verbindungen wie 2-Cyclooctyl-6-tert.butylphenol.

Gegenstand der Erfindung ist daher ein Verfahren zur Herstellung von ortho-substituierten Alkylphenolen mit Katalysatoren erhöhter Aktivität, durch Umsetzung von Phenol oder 2-Alkyl-Phenolen mit 2-Alkyl- bzw. 2-Aryl-1-alkenen in der Flüssigphase, insbesondere in Gegenwart geeigneter inerter Verdünnungsmittel und/oder eines Überschusses an umzusetzendem Alken, bei Temperaturen von 0 ° bis 100 °C und Drücken von 0,1 bis 20 bar, welches dadurch gekennzeichnet ist, daß man die Umsetzung in Gegenwart modifizierter aluminiumhaltiger Katalysatoren durchführt, die durch Zusatz kleiner Mengen als Cokatalysatoren wirkender Polyhalogenphenole mit mindestens drei und vorzugsweise fünf gleichen oder verschiedenen Halogensubstituenten (aus der Gruppe der Elemente Fluor, Chlor und Brom) zu an sich bekannten Aluminiumtrisphenolatkatalysatoren erhalten werden, wobei die Katalysatorformierung in Gegenwart der Cokatalysatoren vorgenommen wird oder die Cokatalysatoren zum bereits formierten Katalysator vor Beginn der Alkylierung in solchen Mengen zugesetzt werden, daß das molare Verhältnis zwischen Cokatalysator und Aluminiumverbindung 0,5 : 1 bis 8 : 1, vorzugsweise 1 : 1 bis 6 : 1, und besonders bevorzugt von 2 : 1 bis 3 : 1, beträgt.

Weiterer Gegenstand der Erfindung sind modifizierte Katalysatoren mit erhöhter Aktivität zur Herstellung von ortho-alkylierten Phenolen, die dadurch gekennzeichnet sind, daß der modifizierte Katalysator

a) aus dem zu alkylierenden (2-Alkyl-)Phenol
b) einem Polyhalogenphenol als Cokatalysator
und
c) einer aluminiumorganischen Verbindung der allgemeinen Formel

$$AlR_{(3-n)}X_n,$$

in der n = 0 oder 1 ist, R für einen Alkylrest mit 1 bis 4 C-Atomen und X für Chlor oder Brom oder Wasserstoff steht, gebildet wird und daß das molare Verhältnis zwischen den Katalysatorkomponenten b) und c) 0,5 bis 8 : 1 und vorzugsweise 2 : 1 bis 3 : 1 beträgt.

Das erfindungsgemäße Verfahren ist sehr vorteilhaft, da der Zusatz der ausgewählten, als Cokatalysatoren dienenden Polyhalogenphenole zu dem zu modifizierenden Aluminiumphenolatkatalysator bzw. vorzugsweise vor dessen Formierung bei der Alkylierung von Phenol oder von gegebenenfalls sekundäre bzw. tertiäre (Cyclo-)Alkylsubstituenten mit 3 bis 10 C-Atomen in der ortho-Position enthaltenden 2-Alkylphenolen mit 2-Alkyl- bzw. 2-Aryl-1-alkenen, insbesondere mit Isobuten, zu einer Erhöhung der Katalysatoraktivität führt. Diese äußert sich in einer Steigerung der Reaktionsgeschwindigkeit bei unveränderter Katalysatoreinsatzmenge und gestattet die Herstellung der z. B. besonders interessierenden 2,6-disubstituierten Phenole in kürzeren Zeiten bzw. mit verringertem Katalysatoreinsatz.

Nach dem vorliegenden Verfahren lassen sich 2,6-Dialkylphenole bei milden Bedingungen (niedrigen Reaktionstemperaturen und -drucken) aus Phenolen oder 2-Alkylphenolen mit 2-Alkyl- bzw. 2-Aryl-1-alkenen (1,1-Dialkylethylenen bzw. 1-Aryl-1-alkylethylenen) in Gegenwart von modifizierten, cokatalysatorhaltigen Aluminium-trisphenolatkatalysatoren in an sich bekannter Weise herstellen, indem

a) die modifizierten Aluminium-trisphenolatkatalysatoren in Mengen von 0,005 bis 5 mol-%, vorzugsweise von 0,05 bis 0,8 mol-% und besonders bevorzugt von 0,1 bis 0,5 mol-%, bezogen auf eingesetztes (2-Alkyl-)Phenol, verwandt werden,

b) die Anlagerung der 2-Alkyl- bzw. 2-Aryl-1-alkene bei Temperaturen von 0 ° bis 100 °C, vorzugsweise von 10 ° bis 80 °C und besonders bevorzugt von 15 ° bis 50 °C sowie bei Drücken von 0,1 bis 20 bar, vorzugsweise von 0,2 bis 6,0 bar und besonders bevorzugt von 0,5 bis 3,5 bar durchgeführt wird,

c) die Umsetzung in der Flüssigphase und vorzugsweise in Gegenwart von gesättigten aliphatischen und/oder cycloaliphatischen Kohlenwasserstoffen, die vorzugsweise 5 bis 10 Kohlenstoffatome enthalten, und/oder von überschüssigem flüssigem bzw. gelöstem Alken erfolgt,

d) die genannten, unter Reaktionsbedingungen flüssigen oder gelösten Verdünnungsmittel in Mengen von 20 bis 1 000 Gewichtsteilen, vorzugsweise von 40 bis 500 Gewichtsteilen und besonders bevorzugt von 60 bis 200 Gewichtsteilen, bezogen auf 100 Gewichsteile (2-Alkyl-)Phenol und die zur Alkylierung verwandten 2-Alkyl- bzw. 2-Aryl-1-alkene in einem molaren Überschuß von 0,2 bis 10 Mol, vorzugsweise von 1 bis 5 Mol und besonders bevorzugt von 1,1 bis 2,5 Mol, bezogen auf 1 Mol (2-Alkyl-)Phenol, zuzusetzen sind bzw. im Falle der Alkene diese in einem Überschuß von 2 bis 10 Mol, vorzugsweise von 2,2 bis 5 Mol, pro Mol (2-Alkyl-)Phenol anwesend sein sollten,

e) die modifizierten Aluminiumtrisphenolatkatalysatoren unter Zusatz von 0,5 bis 8 Mol, vorzugsweise von 1,0 bis 6,0 Mol und besonders bevorzugt von 2,0 bis 3,0 mol Polyhalogenphenol pro Mol des nichtmodifizierten Katalysators im (2-Alkyl-)Phenol vor Beginn der Alkylierung mit den anschließend zuzudosierenden 2-Alkyl- bzw. 2-Aryl-1-alkenen hergestellt werden

sowie

f) als Cokatalysatoren Fluor, Chlor und/oder Brom enthaltende Polyhalogenphenole mit mindestens 3 und vorzugsweise 5 gleichartigen oder verschiedenen Halogensubstituenten in bestimmten, unter e) erwähnten Mengen pro Mol des aluminiumhaltigen nichtmodifizierten Katalysators zu verwenden sind und unter den in Frage kommenden Cokatalysatoren 2,4,6-Trichlorphenol, Pentafluor-, Pentachlor- und Pentabromphenol bevorzugt sind, wobei Pentachlorphenol besonders bevorzugt ist.

Das Molverhältnis zwischen Cokatalysator und Aluminiumphenolatkatalysator kann in den (unter e) angegebenen Grenzen variiert werden. Die jeweils erzielbare Aktivitätserhöhung wird nicht nur durch dieses Molverhältnis, sondern auch durch die Art des verwandten erfindungsgemäßen Cokatalysators und durch die Konstitution des umzusetzenden (2-Alkyl-)Phenols bzw. des anzulagernden Alkens beeinflußt. Im Einzelfall optimale Bedingungen hinsichtlich des Molverhältnisses, der Reaktionstemperatur und des Drukkes sind daher durch Versuche zu ermitteln, wie sie der Fachmann leicht durchführen kann, siehe Beispiele.

Im allgemeinen wird die Katalysatorformierung durch Zusatz der vorgesehenen Menge an Cokatalysator zum vorgelegten, gegebenenfalls in einem Verdünnungsmittel gelösten (2-Alkyl-)Phenol und anschließende Zugabe der aluminiumorganischen Verbindung erfolgen, ehe die eigentliche Alkylierung unter Zudosierung des Alkens begonnen wird. Andererseits kann die Katalysatormodifizierung auch vorgenommen werden, indem man den Cokatalysator auf einen aus (2-Alkyl-)Phenol und Aluminiumverbindungen hergestellten Katalysator einwirken läßt.

Die Temperatur der Katalysatorformierung ist an sich nicht kritisch, doch wird man zweckmäßigerweise die für die Alkylierung vorgesehene Reaktionstemperatur nicht überschreiten und vorzugsweise die aluminiumorganische Komponente bei Raumtemperatur oder darunter zugeben.

Alle zu alkylierenden (2-Alkyl-)Phenole, die verwandten Alkene und die gegebenenfalls benutzten Verdünnungsmittel müssen frei von Wasser und Katalysatorgiften sein. Besonders bevorzugt als Verdünnungsmittel sind gesättigte aliphatische oder cycloaliphatische Kohlenwasserstoffe, wie z. B. Hexan, Cyclohexan, Ethylcyclohexan, Isopropylcyclohexan (Hydrocumol) oder Dekalin. Geeignete 2-Alkyl- bzw. 2-Aryl-1-alkene sind z. B. Isobuten, $\alpha$-Methyl-Styrol u. a.

Die nach dem erfindungsgemäßen Verfahren mit größerer Reaktionsgeschwindigkeit bzw. mit geringerem Katalysatoreinsatz herstellbaren ortho-disubstituierten Phenole stellen Verbindungen dar, aus denen wertvolle Folgeprodukte, beispielsweise auf dem Gebiet der phenolischen Antioxidantien oder der Pharmazeutika, gewonnen werden können.

Das erfindungsgemäße Verfahren wird durch die nachfolgenden Beispiele näher erläutert.

**Beispiele:**

Die Alkylierungen von Phenol bzw. 2-Alkyl-Phenolen wurden mit Isobuten (als Vertreter der 2-Alkyl-1-alkene) in einem Rührautoklaven bei möglichst hoher Rührerdrehzahl (< 1400 Upm) durchgeführt. Kühlung bzw. Temperierung des Reaktors erfolgten mit einem Hochleistungsthermostaten. Ein im Innern des Reaktors angebrachter Thermofühler erlaubte das Registrieren der Reaktionstemperatur. Veränderungen des Reaktorfüllstandes konnten über eine geeignete Meßvorrichtung verfolgt werden. Proben für gaschromatographische Analysen wurden über einen Stutzen am Reaktor entnommen. Ehe die Anteile der verschiedenen Komponenten der Reaktionsgemische gaschromatographisch bestimmt wurden, wurde der in den Proben enthaltene Katalysator durch Zusatz einiger Tropfen Wasser desaktiviert. Gegebenenfalls vorhandene Beimengungen an Lösemitteln wurden bei der Auswertung der Analysen nicht berücksichtigt, d. h. die in den folgenden Tabellen enthaltenen Angaben zu den verschiedenen Beispielen geben die Zusammensetzung (in Gew.-%) von "lösungsmittelfreien" Reaktionsgemischen wieder.
Bei sämtlichen in den Beispielen angeführten Alkylierungen wurden die angegebenen Mengen an (2-Alkyl-)Phenol in dem trockenen Rührautoklaven vorgelegt, gegebenenfalls die angegebenen Mengen der verschiedenen Lösemittel zugesetzt und anschließend die gewünschten Anteile an Katalysator durch Zugabe von Aluminiumtriethyl, gelöst in (cyclo-)aliphatischen Kohlenwasserstoffen, bei maximal 30 °C formiert, nachdem zuvor die vorgesehene Menge an Cokatalysator zugesetzt und Reaktionsgemisch und Reaktor durch Spülen mit Inertgas von Luft (bzw. Sauerstoffresten) befreit worden waren. Nach Entfernen des freigesetzten Ethans wurde der Reaktor verschlossen und sein Inhalt temperiert. Bei der gewünschten Reaktionstemperatur wurde anschließend unter kräftiger Durchmischung Isobuten in der vorgesehenen Menge zudosiert. Die isotherm durchgeführten Alkylierungen wurden im allgemeinen beendet, sobald keine nennenswerte weitere Abnahme des Gehalts an eingesetztem (2-Alkyl-)Phenol zu beobachten war bzw. sobald hohe Anteile an 2-Alkyl-6-tert.butylphenol erreicht waren.

Bedingt durch das in nur kleinen Mengen verwandte Katalysatorsystem und dessen Empfindlichkeit gegen bestimmte Verunreinigungen sind Vergleiche nur innerhalb einer Serie von Versuchen zulässig, bei der gleiche Chargen an Edukt, Lösemittel, Aktivator und Aluminiumtriethyl zum Einsatz kamen und während der Untersuchungen unverändert blieben. Dies wurde durch Kontrollversuche sichergestellt.

In den Tabellen ist stets die eingesetzte Gesamtmenge an (2-Alkyl-)Phenol und an Isobuten angegeben. Bei den in bar angegebenen Drucken handelt es sich um die in System eingestellten Gesamtdrucke (Absolutdrucke).
Soweit nicht anders vermerkt, wurden in den Tabellen die folgenden Abkürzungen verwandt:

| | |
|---|---|
| CoK = Cokatalysator<br>Al = $Al(C_2H_5)_3$ | 2-TBP = 2-Tert.butylphenol<br>2,6-DTBP = 2,6-Ditert.butylphenol<br>2,4,6-TTBP = 2,4,6-Tritert.butylphenol |

Beispiele 1 bis 4 (Beispiel 1 nicht erfindungsgemäß, Tabelle I)

In der zuvor beschriebenen Weise wurden 251 g (1,67 mol) 2-Tert.butylphenol (Abk. 2-TBP) bei 1,5 bar mit 1,9-2,0 mol Isobuten in Gegenwart von 5,8 mmol Aluminium-tris-(2-tert.butylphenolat) sowie in den erfindungsgemäßen Beispielen von jeweils 11,8 mmol der angegebenen Cokatalysatoren bei 10 °C umgesetzt. Die nach den angegebenen Reaktionszeiten entnommenen Proben belegen, daß 2-TBP in Gegenwart der Cokatalysatoren rascher als im Vergleichsversuch verbraucht wird (vgl. Tabelle I) und in 2,6-Ditert.butylphenol (Abk. 2,6-DTBP) sowie - zu einem weit geringerem Teil - in 2,4,6-Tritert.butylphenol (Abk. 2,4,6-TTBP) und andere Nebenprodukte übergeht.

Beispiele 5 bis 18
(Beispiel 5 - nicht erfindungsgemäß, Beispiele 6-13 - erfindungsgemäß, jeweils Tabelle II; Beispiel 14 - nicht erfindungsgemäß sowie Beispiele 15-18 - erfindungsgemäß, jeweils Tabelle III)

In der bei den Beispielen 1 bis 4 angewandten Arbeitsweise wurde 2-Tert.butylphenol (jeweils 1,67 mol = 251 g) nach Zusatz von Cyclohexan (jeweils 200 ml) bei 10 °C und einem Druck von 1,5 bzw. 1,6 bar mit überschüssigem Isobuten (2,1-2,6 mol bei den Versuchen in Tabelle II, 2,7-3,0 mol bei den in Tabelle III enthaltenen Beispielen 14 bis 18) in Gegenwart von 5,8 mmol des aluminiumhaltigen Alkylierungskatalysator umgesetzt. Neben dem Verdünnungsmittel Cyclohexan wurden bei den erfindungsgemäßen Beispielen 6 bis 13 und 15 bis 18 die in den Tabellen II und III aufgeführten Cokatalysatoren in bestimmten Molverhältnissen bezogen auf die vorgesehene Menge an aluminiumhaltigem Katalysator vor der Katalysatorformierung zugesetzt.
Man erkennt, daß die Umsetzung von 2-Tert.butylphenol bei Anwesenheit der Cokatalysatoren auch in Gegenwart des Verdünnungsmittels rascher verläuft, so daß 2,6-Ditert.butylphenol (Abk. 2,6-DTBP) trotz der niedrigen Reaktionstemperatur und der geringen verwandten Katalysatormengen nach dem erfindungsgemäßen Verfahren mit hoher Geschwindigkeit hergestellt werden kann. Durch den Zusatz des Verdünnungsmittels und/ oder die Erhöhung des Isobutenüberschusses werden bei vergleichbaren 2-TBP-Umsätzen und Cokatalysator-Aluminium-Molverhältnissen überwiegend niedrigere Anteile an 2,4,6-Tritert.butylphenol als bei Abwesenheit von Verdünnungsmitteln (vgl. Tabelle I) erhalten, d. h. die für die Wirtschaftlichkeit des Herstellungsverfahrens bedeutsame Selektivität der 2,6-DTBP-Bildung wird erhöht.

Beispiele 19 bis 25 (davon 19 und 23 nicht erfindungsgemäß, Tabelle IV)

Nach der zuvor beschriebenen Verfahrensweise wurden 1,67 mol (251 g) 2-Tert.butylphenol bei 30° bzw. 50 °C mit überschüssigem Isobuten bei 1,7 bar umgesetzt, nachdem das Edukt in der vorgesehenen Menge an Verdünnungsmittel (jeweils 200 ml Cyclohexan) gelöst und gegebenenfalls vor der Katalysatorformierung (durch Zugabe von 5,8 mmol Aluminiumtriethyl) die der Tabelle IV zu entnehmenden Mengen der verschiedenen Cokatalysatoren zugesetzt worden waren.

Der Vergleich der Ergebnisse der erfindungsgemäßen Beispiele 20 bis 22 bzw. 24 und 25 mit den zugehörigen ohne Cokatalysatorzusatz durchgeführten Beispielen 19 bzw. 23 zeigt, wie wirksam die aufgeführten Aktivatoren auch bei den angewandten, gegenüber den Beispielen 1 bis 18 erhöhten Reaktionstemperaturen sind.

Beispiele 26 bis 33 (davon 26,32 und 33 nicht erfindungsgemäß, Tabelle V)

In der bei den Beispielen 1 bis 25 angewandten Arbeitsweise wurden 1,67 mol (251 g) 2-Tert.butylphenol in Gegenwart von 200 ml Cyclohexan nach Zusatz der in Tabelle V angegebenen Mengen fluorhaltiger Verbindungen und nach Katalysatorformierung mit 5,8 mmol Aluminiumtriethyl bei 10 °C und bei einem Druck von 1,5 bar mit überschüssigem Isobuten umgesetzt.

Die Ergebnisse zeigen, sofern man die mit einem nichtmodifizierten Katalysator nach unterschiedlichen Reaktionszeiten erreichte (als Beispiel 26 aufgeführte) Zusammensetzung des Reaktionsgemisches als Bezugsbasis heranzieht, daß bei den erfindungsgemäßen Beispielen 27 bis 31 eine Beschleunigung der Umsetzung zu verzeichnen ist, die sich vor allem bei hohen Umsätzen des 2-TBP bemerkbar macht.
Andererseits belegen die nicht erfindungsgemäßen Beispiele 32 und 33, daß die mit ausgewählten Polyhalogenphenolen erzielbaren Steigerungen der Katalysatoraktivität mit anderen, ähnlichen Verbindungen nicht beobachtet werden, wenn diese in vergleichbaren molaren Mengen anwesend sind. Selbst mit Verbindungen sehr ähnlicher Konstitution kann es bereits bei Zusatz kleiner Mengen auch zu einem Abfall der Katalysatoraktivität kommen.

Beispiele 34 bis 38 (Beispiel 34 nicht erfindungsgemäß, Tabelle VI)

In der bei den Beispielen 1 bis 33 angewandten Arbeitsweise wurden jeweils 0,83 mol (171 g) 2,4-Ditert.butylphenol (Abk. 2,4-DTBP) in 350 ml Hexan gelöst, gegebenenfalls mit den in Tabelle VI angegebenen Mengen verschiedener Polyhalogenphenole sowie anschließend mit 5,8 mmol Aluminiumtriethyl bei Raumtemperatur versetzt und dann mit 1,2 bis 1,6 mol Isobuten bei 30 °C und bei einem Druck von 1,7 bar zu 2,4,6-Tritert.butylphenol (Abk. 2,4,6-TTBP) umgesetzt.
Die in Tabelle VI aufgeführten Zusammensetzungen der nach unterschiedlichen Reaktionszeiten entnommenen Proben zeigen, daß die erfindungsgemäß modifizierten Katalysatoren auch die Alkylierung von 2,4-DTBP mit Isobuten deutlich beschleunigen. Der Wechsel des Verdünnungsmittels beeinträchtigt ihre Wirkung nicht.

Beispiele 39 bis 45 (Beispiele 39 und 41 nicht erfindungsgemäß, Tabelle VII)

In der zuvor beschriebenen Verfahrensweise wurden 0,5 mol (102,2 g) 2-Cyclooctylphenol mit 100 ml Cyclohexan verdünnt und jeweils 1,7 mmol Alkylierungskatalysator im gegebenenfalls die erfindungsgemäß zuzusetzenden Cokatalysatoren enthaltenden Reaktionsgemisch (durch Zugabe von 1,7 mmol Aluminiumtriethyl) formiert, ehe bei den der Tabelle VII zu entnehmenden Temperaturen die Umsetzung des Edukts mit überschüssigem Isobuten zu 2-Cyclooctyl-6-tert.butylphenol bei 1,9 bar vorgenommen wurde. Diese Verbindung war bisher nicht bekannt.
Man erkennt an den in Tabelle VII aufgeführten Gehalten an 2-Cyclooctylphenol, daß die Alkylierung sowohl bei 10 °C als auch bei 30 °C durch Modifizierung des Katalysators mit Pentachlorphenol erheblich beschleunigt werden kann, so daß höhere Anteile des 2,6-Dialkylphenols (2-Cyclooctyl-6-tert.butylphenol) schneller und damit wirtschaftlicher gewonnen werden können.

Beipiele 46 bis 51 (Beispiel 46 nicht erfindungsgemäß, Tabelle VIII)

In der bei den Beispielen 1 bis 45 angewandten Arbeitsweise wurde 2-Cyclohexylphenol bei 30 °C mit überschüssigem Isobuten alkyliert. Die Umsetzungen erfolgten bei einem Druck von 2,2 bar mit den Tabelle VIII zu entnehmenden Mengen an Edukt, Verdünnungsmittel, aluminiumhaltigem gegebenenfalls mit Pentafluor- bzw. Pentachlorphenol modifiziertem Katalysator und Alken.

Die Zusammensetzung der nach verschiedenen Reaktionszeiten entnommenen Proben belegt die vorteilhafte Wirkung der zur Katalysatormodifizierung erfindungsgemäß zuzusetzenden Verbindungen.

Beispiele 52 bis 57 (Beispiele 52 und 55 nicht erfindungsgemäß, Tabelle IX)

Nach der zuvor beschriebenen Verfahrensweise wurden jeweils 1,0 mol 2-Isopropylphenol (136,2 g) bzw. Phenol (94,1 g) in Gegenwart eines durch Zugabe von 3,4 mmol Aluminiumtriethyl erzeugten, gegebenenfalls mit Pentachlorphenol in der der Tabelle IX zu entnehmenden Menge modifizierten Katalysators mit überschüssigem Isobuten bei 50 ° bzw. 10 °C alkyliert.
Obwohl die Umsetzung von Isopropylphenol und die unter Verdünnung mit Cyclohexan durchgeführte von Phenol bei 50 °C langsam verlaufen, bewirkt die Anwesenheit von Pentachlorphenol als Cokatalysator doch eine signifikante Beschleunigung.

**Tabelle I:**

| Beispiel | Cokatalysator | Molverh. CoK/Al | Reaktionszeit [min] | Zusammensetzung des Rkt.-gemisches (% lt. GC) 2-TBP** | 2,6-DTBP | 2,4,6-TTBP |
|---|---|---|---|---|---|---|
| 1 | - (Vergleichsversuch) | - | 30 | 46,5 | 50,0 | 2,1 |
| | | | 40 | 34,7 | 61,2 | 2,8 |
| | | | 60 | 19,9 | 75,5 | 3,8 |
| | | | 75 | 13,3 | 81,9 | 4,1 |
| | | | 90 | 9,1 | 86,0 | 4,3 |
| | | | 120 | 5,2 | 89,3 | 4,8 |
| | | | 180 | 2,1 | 92,5 | 4,9 |
| 2 | 2,4,6-Trichlorphenol (TCP) | 2:1 | 30 | 40,0 | 55,6 | 2,5 |
| | | | 40 | 28,0 | 67,0 | 3,3 |
| | | | 60 | 13,2 | 81,7 | 4,0 |
| | | | 75 | 6,0 | 88,7 | 4,4 |
| | | | 90 | 2,1 | 92,7 | 4,5 |
| | | | 120 | 0,7 | 93,7 | 4,7 |
| | | | 180 | 0,7 | 93,7 | 4,9 |
| 3 | 2,4,6-Tribromphenol (TBRP) | | 30 | 39,4 | 55,9 | 2,9 |
| | | | 40 | 26,6 | 67,9 | 3,8 |
| | | | 60 | 10,7 | 83,3 | 4,8 |
| | | | 75 | 2,8 | 90,9 | 5,2 |
| | | | 90 | 1,3 | 92,5 | 5,3 |
| | | | 120 | 1,1 | 92,6 | 5,5 |
| | | | 180 | 1,0 | 92,2 | 5,9 |
| 4 | Pentachlorphenol (PCP) | | 30 | 40,5 | 52,8 | 4,4 |
| | | | 40 | 24,8 | 66,7 | 6,5 |
| | | | 60 | 6,2 | 83,8 | 9,0 |
| | | | 75 | 0,8 | 88,9 | 9,3 |
| | | | 90 | 0,5 | 88,7 | 9,9 |
| | | | 120 | 0,5 | 88,2 | 10,2 |
| | | | 180 | 0,5 | 87,4 | 11,1 |

* Erläuterungen: Alle Ansätze unter Einsatz von 1,67 mol (251 g) 2-TBP, 5,8 mmol $Al(C_2H_5)_3$ (1,02 molar in Hexan) und 1,9 - 2,0 mol Isobuten bei 1,5 bar.

** Abk.:
CoK = Cokatalysator
Al = $Al(C_2H_5)_3$
TCP = 2,4,6-Trichlorphenol
TBRP = 2,4,6-Tribromphenol
PCP = Pentachlorphenol
2-TBP = 2-Tert.butylphenol
2,6-DTBP = 2,6-Ditert.butylphenol
2,4,6-TTBP = 2,4,6-Tritert.butylphenol

**Tabelle II:** **Alkylierung von 2-Tert.butylphenol mit Isobuten bei 10 °C in Gegenwart verschiedener Polyhalogenphenole*** **(Versuche unter Zusatz eines Verdünnungsmittels)**

| Beispiel | Cokatalysator | Molverh. CoK/Al | Reaktionszeit [min] | Zusammensetzung des Rkt.-gemisches (% lt. GC) 2-TBP | 2,6-DTBP | 2,4,6-TTBP |
|---|---|---|---|---|---|---|
| 5 | - (Vergleichsversuch) | - | 30 | 59,2 | 39,9 | 0,4 |
| | | | 40 | 48,2 | 50,6 | 0,6 |
| | | | 60 | 34,0 | 64,6 | 0,9 |
| | | | 75 | 25,9 | 73,0 | 1,1 |
| | | | 90 | 21,5 | 77,0 | 1,2 |
| | | | 120 | 14,3 | 83,9 | 1,4 |
| | | | 180 | 7,7 | 90,4 | 1,6 |
| 6 | 2,4,6-Trichlorphenol | 2:1 | 30 | 55,8 | 42,7 | 0,4 |
| | | | 40 | 45,9 | 52,6 | 0,6 |
| | | | 60 | 30,8 | 67,3 | 0,9 |
| | | | 75 | 21,4 | 76,6 | 1,1 |
| | | | 90 | 17,2 | 80,9 | 1,2 |
| | | | 120 | 9,6 | 88,4 | 1,4 |
| | | | 180 | 3,5 | 94,2 | 1,6 |
| 7 | | 3:1 | 30 | 51,6 | 47,0 | 0,5 |
| | | | 40 | 39,1 | 58,7 | 0,7 |
| | | | 60 | 24,0 | 74,1 | 1,0 |
| | | | 75 | 14,6 | 83,3 | 1,2 |
| | | | 90 | 10,5 | 87,3 | 1,3 |
| | | | 120 | 4,1 | 93,6 | 1,5 |
| | | | 180 | 1,3 | 96,5 | 1,5 |
| 8 | | 4:1 | 30 | 59,6 | 38,7 | 0,3 |
| | | | 40 | 49,3 | 49,1 | 0,5 |
| | | | 60 | 33,2 | 64,8 | 0,8 |
| | | | 75 | 23,4 | 74,4 | 1,0 |
| | | | 90 | 17,1 | 80,6 | 1,2 |
| | | | 120 | 8,6 | 89,0 | 1,4 |
| | | | 180 | 2,3 | 95,2 | 1,6 |
| ===== | ============= | ========= | ========== | ========= | ========= | ========= |
| 9 | 2,4,6-Tribromphenol | 2:1 | 30 | 59,2 | 39,6 | 0,4 |
| | | | 60 | 33,0 | 65,3 | 1,0 |
| | | | 75 | 24,4 | 73,5 | 1,2 |
| | | | 90 | 18,4 | 79,5 | 1,3 |
| | | | 120 | 9,1 | 88,6 | 1,6 |
| | | | 180 | 1,7 | 95,9 | 1,8 |

**Tabelle II:** - Fortsetzung -

| Beispiel | Cokatalysator | Molverh. CoK/Al | Reaktionszeit [min] | Zusammensetzung des Rkt.-gemisches (% lt. GC) 2-TBP | 2,6-DTBP | 2,4,6-TTBP |
|---|---|---|---|---|---|---|
| 10 | | 3:1 | 30 | 64,2 | 34,4 | 0,4 |
| | | | 60 | 40,1 | 57,9 | 0,8 |
| | | | 75 | 29,6 | 68,3 | 1,1 |
| | | | 90 | 22,6 | 75,0 | 1,3 |
| | | | 120 | 13,2 | 84,6 | 1,5 |
| | | | 180 | 2,7 | 94,6 | 1,9 |
| 11 | Pentachlorphenol | 1:1 | 30 | 46,3 | 52,2 | 0,8 |
| | | | 40 | 35,2 | 63,0 | 1,1 |
| | | | 60 | 19,2 | 78,8 | 1,6 |
| | | | 75 | 9,9 | 87,6 | 2,0 |
| | | | 90 | 5,5 | 91,8 | 2,3 |
| | | | 120 | 1,2 | 95,7 | 2,7 |
| | | | 180 | 0,8 | 95,9 | 2,9 |
| 12 | | 2:1 | 30 | 40,8 | 57,0 | 1,3 |
| | | | 40 | 24,6 | 72,7 | 2,0 |
| | | | 60 | 4,5 | 91,8 | 3,2 |
| | | | 75 | 0,6 | 95,2 | 3,7 |
| | | | 90 | 0,7 | 95,0 | 3,7 |
| | | | 120 | 0,8 | 94,5 | 4,2 |
| | | | 180 | 0,6 | 94,0 | 4,9 |
| | | | 360 | 0,6 | 92,2 | 6,6 |
| 13 | | 3:1 | 30 | 24,7 | 71,8 | 2,9 |
| | | | 40 | 9,1 | 86,1 | 4,0 |
| | | | 60 | 0,6 | 93,1 | 5,6 |
| | | | 75 | 0,4 | 92,1 | 6,6 |
| | | | 90 | 0,4 | 91,5 | 7,2 |
| | | | 120 | 0,5 | 90,3 | 8,4 |
| | | | 180 | 0,4 | 88,1 | 10,7 |
| | | | 360 | 0,6 | 81,9 | 16,2 |

* Erläuterungen: Einsatz jeweils 1,67 mol (251 g) 2-TBP, 5,8 mmol $Al(C_2H_5)_3$ 200 ml Cyclohexan und 2,1-2,6 mol Isobuten bei 10 °C/1,5 bar

** Abk.: siehe Tabelle I

**Tabelle III:** Alkylierung von 2-Tert.butylphenol mit Isobuten bei 10 °C in Gegenwart verschiedener Polyhalogenphenole*
**(Versuche unter Zusatz eines Verdünnungsmittels)**

| Beispiel | Cokatalysator | Molverh. CoK/Al | Reaktionszeit [min] | Zusammensetzung des Rkt.-gemisches (% lt. GC) 2-TBP** | 2,6-DTBP | 2,4,6-TTBP |
|---|---|---|---|---|---|---|
| 14 | - (Vergleichsversuch) | - | 30 | 65,2 | 34,0 | 0,4 |
| | | | 60 | 38,6 | 60,1 | 0,8 |
| | | | 75 | 30,1 | 68,6 | 1,0 |
| | | | 90 | 23,6 | 74,8 | 1,1 |
| | | | 120 | 14,1 | 84,5 | 1,3 |
| | | | 180 | 4,8 | 93,3 | 1,6 |
| | | | 360 | 0,5 | 97,4 | 1,7 |
| 15 | 2,4,6-Tribromphenol | 2:1 | 30 | 66,4 | 32,9 | 0,3 |
| | | | 60 | 38,8 | 59,8 | 0,8 |
| | | | 75 | 30,3 | 68,3 | 1,0 |
| | | | 90 | 20,8 | 77,5 | 1,2 |
| | | | 120 | 7,8 | 90,4 | 1,5 |
| | | | 180 | 0,2 | 97,6 | 1,9 |
| | | | 360 | 0,1 | 97,2 | 2,5 |
| 16 | | 4:1 | 30 | 72,8 | 26,5 | 0,2 |
| | | | 60 | 48,0 | 50,8 | 0,7 |
| | | | 75 | 35,5 | 62,9 | 0,9 |
| | | | 90 | 25,8 | 72,3 | 1,2 |
| | | | 120 | 12,4 | 85,5 | 1,6 |
| | | | 180 | 1,5 | 95,7 | 2,2 |
| | | | 360 | - | 92,5 | 7,5 |
| 17 | Pentabromphenol | 2:1 | 30 | 66,0 | 32,7 | 0,4 |
| | | | 40 | 54,9 | 43,8 | 0,7 |
| | | | 60 | 35,8 | 62,2 | 1,3 |
| | | | 75 | 26,3 | 71,4 | 1,6 |
| | | | 90 | 16,2 | 81,2 | 2,1 |
| | | | 120 | 3,2 | 93,6 | 2,9 |
| | | | 180 | 0,3 | 95,8 | 3,5 |
| | | | 360 | 0,1 | 95,0 | 4,5 |
| 18 | | 4:1 | 30 | 67,1 | 31,9 | 0,5 |
| | | | 40 | 58,2 | 40,6 | 0,7 |
| | | | 60 | 37,8 | 60,1 | 1,3 |
| | | | 75 | 24,4 | 73,0 | 1,9 |
| | | | 90 | 13,7 | 83,0 | 2,5 |
| | | | 120 | 2,4 | 93,5 | 3,3 |
| | | | 180 | 0,1 | 95,0 | 4,5 |
| | | | 360 | 0,2 | 89,8 | 9,6 |

* Erläuterungen: Einsatz jeweils 1,67 mol (251 g) 2-TBP, 5,8 mmol $Al(C_2H_5)_3$, 200 ml Cyclohexan und 2,7 - 3,0 mol Isobuten bei 10 °C/1,6 bar

** Abk.: siehe Tabelle I

**Tabelle IV: Alkylierung von 2-Tert.butylphenol mit Isobuten in Gegenwart verschiedener Polyhalogenphenole* (Versuche mit Cyclohexan als Verdünnungsmittel bei 30 ° bzw. 50 °C)**

| Beispiel | Cokatalysator | Molverh. CoK/Al | Reaktions-temp. [°C] | zeit [min] | Zusammensetzung des Rkt.-gemisches (% lt. GC) 2-TBP** | 2,6-DTBP | 2,4,6-TTBP |
|---|---|---|---|---|---|---|---|
| 19 | - (Vergleichsversuch) | - | 30° | 30 | 45,7 | 52,2 | 1,1 |
| | | | | 60 | 20,7 | 76,7 | 1,9 |
| | | | | 75 | 14,8 | 82,6 | 2,1 |
| | | | | 90 | 10,5 | 85,6 | 2,6 |
| | | | | 120 | 5,5 | 91,7 | 2,5 |
| | | | | 180 | 2,7 | 94,0 | 2,8 |
| 20 | 2,4,6-Trichlorphenol | 5:1 | 30° | 30 | 30,4 | 66,7 | 2,0 |
| | | | | 60 | 5,6 | 90,6 | 3,0 |
| | | | | 75 | 2,7 | 93,1 | 3,4 |
| | | | | 90 | 1,1 | 93,7 | 3,9 |
| | | | | 120 | 0,2 | 93,6 | 4,9 |
| | | | | 180 | 0,2 | 91,3 | 7,6 |
| 21 | Pentachlorphenol | | | 30 | 5,8 | 75,1 | 15,8 |
| | | | | 60 | 0,2 | 67,3 | 30,4 |
| | | | | 75 | 0,2 | 62,6 | 35,4 |
| | | | | 90 | 0,2 | 59,5 | 38,0 |
| | | | | 120 | 0,2 | 56,1 | 41,1 |
| | | | | 180 | 0,3 | 54,8 | 43,0 |
| 22 | Pentafluorphenol | 3,5:1 | | 30 | 3,4 | 80,3 | 13,2 |
| | | | | 60 | 0,4 | 53,3 | 43,9 |
| | | | | 75 | 0,5 | 52,8 | 43,8 |
| | | | | 90 | 0,5 | 51,5 | 44,4 |
| | | | | 120 | 0,7 | 50,6 | 43,0 |
| | | | | 180 | 0,9 | 49,5 | 45,1 |
| 23 | - (Vergleichsversuch) | - | 50° | 30 | 53,2 | 43,9 | 1,4 |
| | | | | 60 | 27,1 | 68,5 | 3,0 |
| | | | | 75 | 21,6 | 74,1 | 3,2 |
| | | | | 90 | 15,6 | 79,9 | 3,5 |
| | | | | 120 | 9,3 | 85,9 | 3,9 |
| | | | | 180 | 4,1 | 90,9 | 4,3 |
| 24 | 2,4,6-Trichlorphenol | 2:1 | | 30 | 55,6 | 41,7 | 1,0 |
| | | | | 60 | 28,8 | 67,1 | 2,4 |
| | | | | 75 | 20,1 | 75,6 | 2,9 |
| | | | | 90 | 13,4 | 82,0 | 3,3 |
| | | | | 120 | 7,5 | 87,1 | 3,4 |
| | | | | 180 | 2,6 | 92,3 | 4,2 |

**Tabelle IV:** - Fortsetzung -

| Bei-spiel | Cokatalysator | Molverh. CoK/Al | Reaktions-temp. [°C] | zeit [min] | Zusammensetzung des Rkt.-gemisches (% lt. GC) 2-TBP** | 2,6-DTBP | 2,4,6-TTBP |
|---|---|---|---|---|---|---|---|
| 25 | Pentachlor-phenol | 2:1 | 50° | 30 | 41,9 | 51,0 | 3,8 |
| | | | | 60 | 11,6 | 77,0 | 8,9 |
| | | | | 75 | 5,4 | 81,6 | 10,9 |
| | | | | 90 | 2,1 | 82,3 | 13,6 |
| | | | | 120 | 0,9 | 81,7 | 15,4 |
| | | | | 180 | 0,6 | 79,0 | 18,4 |

* Erläuterungen: Alle Ansätze unter Einsatz von 1,67 mol (251 g) 2-TBP, 5,8 mmol $Al(C_2H_5)_3$ (1,02 molar in Hexan), 200 mol Cyclohexan und 1,8-2,1 mol Isobuten bei 1,7 bar

** Abk.: siehe Tabelle I

Tabelle V: Alkylierung von 2-Tert.butylphenol mit Isobuten in Gegenwart fluorhaltiger Verbindungen*
(Versuche bei 10 °C mit Cyclohexan als Verdünnungsmittel)

| Beispiel Nr. | Cokatalysator | Molverh. CoK/Al | Reaktionszeit [min] | Zusammensetzung des Rkt.-gemisches (% lt. GC) 2-TBP** | 2,6-DTBP | 2,4,6-TTBP |
|---|---|---|---|---|---|---|
| 26 | (Vergleichsversuch) | - | 30 | 45,9 | 52,8 | 0,7 |
| | | | 60 | 22,7 | 75,6 | 1,2 |
| | | | 75 | 16,9 | 81,3 | 1,4 |
| | | | 90 | 12,3 | 85,8 | 1,5 |
| | | | 120 | 6,9 | 91,1 | 1,6 |
| | | | 180 | 3,0 | 94,9 | 1,8 |
| 27 | Pentafluorphenol | 1:1,3 | 30 | 32,4 | 64,9 | 1,2 |
| | | | 60 | 7,3 | 88,9 | 2,2 |
| | | | 75 | 3,2 | 92,4 | 2,8 |
| | | | 90 | 1,7 | 93,5 | 3,1 |
| | | | 120 | 0,8 | 93,7 | 3,9 |
| | | | 180 | 0,6 | 93,3 | 4,5 |
| | | | 360 | 0,4 | 92,6 | 5,1 |
| 28 | | 1:2 | 30 | 26,5 | 70,3 | 1,4 |
| | | | 60 | 4,0 | 91,3 | 2,8 |
| | | | 75 | 1,4 | 93,4 | 3,4 |
| | | | 90 | 0,9 | 93,5 | 3,8 |
| | | | 120 | 0,6 | 93,4 | 4,2 |
| | | | 180 | 0,6 | 92,8 | 4,9 |
| 29 | | 1:3 | 30 | 26,5 | 69,0 | 1,7 |
| | | | 60 | 2,8 | 91,0 | 3,7 |
| | | | 75 | 0,7 | 92,7 | 4,7 |
| | | | 120 | 0,5 | 90,6 | 7,4 |
| | | | 180 | 0,4 | 90,5 | 7,8 |
| 30 | 2,4-Dichlor-4-fluorphenol | 1:1 | 30 | 48,0 | 50,6 | 0,6 |
| | | | 60 | 22,0 | 76,2 | 1,1 |
| | | | 75 | 14,6 | 83,5 | 1,3 |
| | | | 90 | 10,3 | 87,8 | 1,4 |
| | | | 120 | 5,6 | 92,3 | 1,5 |
| | | | 180 | 2,0 | 95,9 | 1,7 |
| | | | 360 | 0,9 | 96,8 | 1,8 |
| 31 | | 2:1 | 30 | 47,0 | 51,6 | 0,5 |
| | | | 60 | 21,5 | 76,8 | 1,0 |
| | | | 75 | 14,7 | 83,5 | 1,1 |
| | | | 90 | 10,0 | 88,1 | 1,2 |
| | | | 120 | 4,7 | 93,4 | 1,3 |
| | | | 180 | 0,8 | 97,1 | 1,6 |
| | | | 360 | 0,7 | 97,2 | 1,6 |

Tabelle V: - Fortsetzung -

| Beispiel Nr. | Cokatalysator | Molverh. CoK/Al | Reaktionszeit [min] | Zusammensetzung des Rkt.-gemisches (% lt. GC) 2-TBP** | 2,6-DTBP | 2,4,6-TTBP |
|---|---|---|---|---|---|---|
| 32 | Pentafluorbenzylalkohol (Vergleichsbeispiel) | 1:1 | 30 | 78,5 | 20,7 | 0,1 |
| | | | 60 | 61,0 | 37,9 | 0,4 |
| | | | 90 | 49,3 | 49,3 | 0,6 |
| | | | 120 | 40,0 | 58,4 | 0,8 |
| | | | 180 | 28,8 | 69,3 | 1,1 |
| 33 | | 1:2 | 30 | 94,1 | 5,2 | 0,0 |
| | | | 60 | 89,0 | 10,2 | 0,0 |
| | | | 120 | 81,2 | 18,0 | 0,1 |
| | | | 180 | 74,0 | 25,0 | 0,2 |
| | | | 360 | 59,4 | 39,4 | 0,3 |

* Erläuterungen: Alle Ansätze unter Einsatz von 1,67 mol (251 g) 2-TBP, 5,8 mmol $Al(C_2H_5)_3$ (1,02 molar in Hexan), 200 ml Cyclohexan und 2,1 - 2,5 mol Isobuten bei 10 °C/1,5 bar.

** Abk.: s. Tabelle I

Tabelle VI: Umsetzung von 2,4-Ditert.butylphenol mit Isobuten bei 30 °C in Gegenwart verschiedener Cokatalysatoren*

| Beispiel Nr. | Cokatalysator | Molverh. CoK/Al | Reaktionszeit [min] | Zusammensetzung des Rkt.-gemisches (% lt. GC) 2,4-DTBP** | 2,4,6-TTBP |
|---|---|---|---|---|---|
| 34 | - (Vergleichsversuch) | - | 30 | 85,6 | 14,3 |
| | | | 60 | 69,2 | 30,8 |
| | | | 90 | 55,1 | 44,3 |
| | | | 120 | 43,8 | 56,0 |
| | | | 180 | 31,3 | 68,6 |
| | | | 360 | 12,7 | 87,1 |
| 35 | 2,4,6-Trichlorphenol | 2:1 | 30 | 81,4 | 18,1 |
| | | | 60 | 59,9 | 39,7 |
| | | | 90 | 43,6 | 55,9 |
| | | | 120 | 31,6 | 68,9 |
| | | | 180 | 17,8 | 81,6 |
| | | | 360 | 2,1 | 97,2 |
| 36 | Pentafluorphenol | 2:1 | 10 | 68,4 | 30,2 |
| | | | 20 | 44,3 | 53,6 |
| | | | 30 | 26,1 | 71,7 |
| | | | 45 | 6,8 | 91,2 |
| | | | 60 | 0,7 | 98,9 |
| | | | 120 | 0,6 | 99,1 |
| 37 | Pentachlorphenol | 1:1 | 20 | 72,1 | 27,7 |
| | | | 30 | 57,0 | 42,9 |
| | | | 45 | 37,6 | 62,1 |
| | | | 60 | 22,6 | 77,1 |
| | | | 90 | 8,6 | 91,1 |
| | | | 120 | 3,1 | 96,7 |
| | | | 180 | 0,9 | 98,9 |
| 38 | | 2:1 | 10 | 80,2 | 19,7 |
| | | | 20 | 52,8 | 47,0 |
| | | | 30 | 27,8 | 72,0 |
| | | | 45 | 6,8 | 93,0 |
| | | | 60 | 1,3 | 98,5 |
| | | | 90 | 0,4 | 99,3 |

* Erläuterungen: Alle Ansätze unter Einsatz von 0,83 mol (171 g) 2,4-DTBP, 4,2 mmol $Al(C_2H_5)_3$ (1,02 molar in Hexan), 350 ml Hexan und 1,2 - 1,6 mol Isobuten bei 30 °C/ 1,7 bar.

** Abk.: s. auch Tabelle I
2,4-DTBP = 2,4-Ditert.butylphenol
2,4,6-TTBP = 2,4,6-Tritert.butylphenol

Tabelle VII: Alkylierung von 2-Cyclooctylphenol mit Isobuten in Gegenwart Pentachlorphenol enthaltender Katalysatoren*

| Beispiel Nr. | Molverhältnis CoK/Al | Reaktions-temp. [°C] | zeit [Stdn.] | Zusammensetzung des Reaktionsgemisches (% lt. GC) 2-COP** | X1 | 2,6-Add. | X2 |
|---|---|---|---|---|---|---|---|
| 39 | - (Vergleichsversuch) | 10° | 2 | 95,3 | 2,0 | 2,6 | |
| | | | 4 | 93,9 | 2,5 | 3,5 | |
| | | | 6 | 89,4 | 4,8 | 5,7 | |
| 40 | 2 : 1 | | 1 | 94,1 | 1,9 | 3,9 | |
| | | | 2 | 89,4 | 3,6 | 7,0 | |
| | | | 4 | 79,8 | 7,0 | 13,2 | |
| | | | 6 | 70,4 | 10,2 | 19,1 | |
| 41 | - (Vergleichsversuch) | 30° | 1 | 95,7 | 1,9 | 2,2 | |
| | | | 2 | 91,9 | 3,7 | 4,4 | |
| | | | 4 | 84,7 | 7,0 | 8,1 | |
| | | | 6 | 77,4 | 10,3 | 12,2 | |
| 42 | 1 : 1 | | 1 | 93,2 | 2,7 | 3,9 | |
| | | | 2 | 87,0 | 5,4 | 7,6 | |
| | | | 4 | 76,1 | 9,8 | 13,9 | |
| | | | 6 | 66,5 | 13,5 | 19,9 | |
| 43 | 2 : 1 | | 1 | 86,9 | 4,8 | 8,4 | |
| | | | 2 | 76,7 | 8,4 | 14,8 | |
| | | | 4 | 57,7 | 14,7 | 27,6 | |
| | | | 6 | 39,9 | 20,1 | 39,8 | 0,1 |
| 44 | 3 : 1 | | 1 | 83,3 | 5,7 | 10,8 | |
| | | | 2 | 70,9 | 8,3 | 20,6 | |
| | | | 4 | 41,6 | 19,1 | 39,2 | |
| | | | 6 | 19,7 | 23,6 | 56,1 | 0,4 |
| 45 | 4 : 1 | | 1 | 79,1 | 6,6 | 14,1 | |
| | | | 2 | 61,6 | 11,7 | 26,5 | 0,1 |
| | | | 4 | 29,3 | 20,8 | 49,3 | 0,3 |
| | | | 6 | 8,7 | 21,6 | 68,8 | 0,8 |

* Erläuterungen: Einsatz jeweils 0,5 mol (102,2 g) 2-Cyclooctylphenol, 1,7 mmol $Al(C_2H_5)_3$, 100 ml Cyclohexan und 1,3 mol (bei 10 °C/1,9 bar) bzw. 0,7-0,8 mol (bei 30 °C/1,9 bar) Isobuten

** Abk.: s. auch Tabelle I
2-COP = 2-Cyclooctylphenol
2,6-Add. = 2-Cyclooctyl-6-tert.butylphenol
$X_1$ = Tert.butylether des 2-COP
$X_2$ = 2-Cyclooctyl-4,6-ditert.butylphenol

Tabelle VIII: Alkylierung von 2-Cyclohexylpehnol mit Isobuten bei 30 °C in Gegenwart modifizierter Katalysatoren*

| Beispiel Nr. | Cokatalysator | Molverh. CoK/Al | Reaktionszeit [min] | Zusammensetzung des Rkt.-gemisches (% lt. GC) 2-CHP** | X1 | CHTBP | X2 |
|---|---|---|---|---|---|---|---|
| 46 | - (Vergleichsversuch) | - | 1 | 96,0 | 1,7 | 2,1 | |
| | | | 2 | 93,4 | 3,1 | 3,1 | |
| | | | 3 | 88,9 | 4,8 | 5,0 | |
| | | | 4 | 87,8 | 6,0 | 5,9 | |
| | | | 6 | 82,6, | 8,4 | 8,8 | |
| | | | M01 | 61,8 | 18,2 | 19,5 | |
| 47 | Pentafluorphenol | 3,5 : 1 | 1 | 79,5 | 9,6 | 10,0 | |
| | | | 2 | 73,2 | 12,6 | 13,3 | |
| | | | 4 | 64,1 | 17,2 | 17,5 | |
| | | | 6 | 56,7 | 19,7 | 21,3 | |
| | | | M01 | 33,3 | 31,1 | 34,0 | |
| 48 | Pentachlorphenol | 1 : 1 | 1 | 93,6 | 2,7 | 3,1 | |
| | | | 2 | 87,8 | 5,3 | 6,0 | |
| | | | 4 | 77,1 | 10,4 | 11,5 | |
| | | | 6 | 68,3 | 14,5 | 16,4 | |
| | | | M01 | 36,3 | 26,8 | 36,2 | |
| 49 | | 2 : 1 | 1 | 90,4 | 4,0 | 5,5 | |
| | | | 2 | 81,7 | 8,0 | 10,2 | |
| | | | 4 | 64,1 | 15,4 | 20,3 | |
| | | | 6 | 47,0 | 20,8 | 28,0 | |
| | | | M01 | 8,2 | 29,6 | 60,1 | 1,9 |
| 50 | | 4 : 1 | 1 | 86,7 | 5,2 | 8,0 | |
| | | | 2 | 73,0 | 10,7 | 16,3 | |
| | | | 4 | 48,1 | 19,8 | 31,6 | |
| | | | 6 | 28,3 | 24,3 | 46,7 | 0,5 |
| | | | M01 | 0,8 | 3,5 | 87,3 | 8,1 |
| 51 | | 6 : 1 | 1 | 82,3 | 6,7 | 10,8 | |
| | | | 2 | 65,1 | 12,7 | 21,6 | |
| | | | 4 | 29,3 | 23,8 | 45,8 | 0,4 |
| | | | 6 | 4,9 | 24,1 | 69,1 | 1,5 |
| | | | M01 | 0,1 | 0,3 | 28,8 | 69,8 |

* Erläuterungen: Alle Ansätze unter Einsatz von 0,5 mol (88,2 g) 2-Cyclohexylphenol, 1,7 mmol $Al(C_2H_5)_3$ (1,02 molar in Cyclohexan), 150 ml Cyclohexan und 0,85-1,0 mol Isobuten bei 30 °C/2,2 bar

** Abk.:
CoK = Cokatalysator
Al = $Al(C_2H_5)_3$
M01 = Probe nach 6 h bei 30 °C zzgl. 16 h bei 20-25 °C
2-CHP = 2-Cyclohexylphenol
CHTBP = 2-Cyclohexyl-6-tert.butylphenol
$X_1$ = Tert.butylether des Edukts
$X_2$ = höhersiedende Komponente (vermutlich Trisubstitutionsprodukt)

Tabelle IX: Alkylierung von 2-Isopropylphenol bzw. Phenol mit Isobuten in Gegenwart pentachlorphenolhaltiger Katalysatoren bei 50 °C (bzw. 10 °C)

| Bei-spiel Nr. | Edukt | Molverh. CoK/Al | Reaktions-temp. [°C] | zeit [Stdn | Zusammensetzung des Reakt.-gemisches (% lt. GC) Edukt | $X_1$* | 2,6-Add. | $X_5$ |
|---|---|---|---|---|---|---|---|---|
| 52 | 2-Isopropyl-phenol (*1) | - (Vergleichs-versuch) | 50° | 1 | 51,1 | 18,1 | 30,3 | 0,2 |
| | | | | 2 | 35,3 | 20,5 | 43,4 | 0,4 |
| | | | | 4 | 22,5 | 19,5 | 60,0 | 0,7 |
| | | | | 6 | 17,1 | 15,7 | 65,5 | 0,9 |
| 53 | | 2:1 | | 0,8 | 24,5 | 17,5 | 56,4 | 0,9 |
| | | | | 1,9 | 5,3 | 7,3 | 83,6 | 2,9 |
| | | | | 4 | 0,2 | 0,2 | 90,5 | 8,3 |
| | | | | 6 | 0,1 | 0,1 | 87,1 | 12,1 |
| 54 | | 2:1 | 10° | 1 | 81,9 | 6,7 | 11,1 | |
| | | | | 2 | 68,6 | 11,7 | 19,4 | 0,1 |
| | | | | 4 | 45,5 | 20,4 | 33,6 | 0,2 |
| | | | | 6 | 29,6 | 27,0 | 42,9 | 0,3 |
| | | | | | Edukt | $Y_1$ | 2-TBP | 2,6-DTBP |
| 55 | Phenol (*2) | - (Vergleichs-versuch) | 50° | 1 | 70,8 | 22,5 | 6,7 | |
| | | | | 2 | 58,8 | 31,4 | 9,6 | 0,1 |
| | | | | 4 | 46,3 | 41,6 | 11,8 | 0,2 |
| | | | | 6 | 37,5 | 48,8 | 13,0 | 0,3 |
| 56 | | 2:1 | | 1 | 69,9 | 22,7 | 7,3 | |
| | | | | 2 | 55,2 | 34,1 | 10,0 | 0,1 |
| | | | | 4 | 43,7 | 42,8 | 12,8 | 0,2 |
| | | | | 6 | 35,0 | 49,0 | 14,9 | 0,4 |
| 57 | | 4:1 | | 1 | 64,2 | 27,0 | 8,6 | 0,1 |
| | | | | 2 | 49,7 | 38,1 | 11,8 | 0,2 |
| | | | | 4 | 33,9 | 50,4 | 15,0 | 0,4 |

Erläuterungen: Einsatz jeweils 1,0 ml 2-Isopropylphenol (136,2 g) bzw. Phenol (94,1 g), 3,4 mmol Al $(C_2H_5)_3$ (1,02 m-Hexanlsg.) sowie
*1 0,9 - 1,5 mol Isobuten bei 50 °C/2,9 bar (bzw. 10 °C/2,2 bar)
*2 1,5 mol Isobuten und 100 ml Cyclohexan bei 50 °C/ 2,8 bar

Abk.: s. auch Tab. I
2,6-Add. = 2-Isopropyl-6-tert.butylphenol
2-TBP = 2-Tert.butylphenol
2,6-DTBP = 2,6-Ditert.butylphenol
$X_1$ bzw. $Y_1$ = Tert.butylether des Edukts
$X_5$ = höhersiedende Komponente (vermutliche Trisubstitutionsprodukt)

## Patentansprüche

1. Verfahren zur Herstellung von ortho-substituierten Alkylphenolen mit Katalysatoren erhöhter Aktivität, durch Umsetzung von Phenol oder 2-Alkyl-Phenolen mit 2-Alkyl- bzw. 2-Aryl-1-alkenen in der Flüssigphase, insbesondere in Gegenwart geeigneter inerter Verdünnungsmittel und/oder eines Überschusses an umzusetzendem Alken, bei Temperaturen von 0 ° bis 100 °C und Drücken von 0,1 bis 20 bar, dadurch gekennzeichnet,
daß man die Umsetzung in Gegenwart modifizierter aluminiumhaltiger Katalysatoren durchführt, die durch Zusatz kleiner Mengen als Cokatalysatoren wirkender Polyhalogenphenole mit mindestens drei und vorzugsweise fünf gleichen oder verschiedenen Halogensubstituenten (aus der Gruppe der Elemente Fluor, Chlor und Brom) zu an sich bekannten Aluminiumtrisphenolatkatalysatoren erhalten werden, wobei die Katalysatorformierung in Gegenwart der Cokatalysatoren vorgenommen wird oder die Cokatalysatoren zum bereits formierten Katalysator vor Beginn der Alkylierung in solchen Mengen zugesetzt werden, daß das molare Verhältnis zwischen Cokatalysator und Aluminiumverbindung 0,5 : 1 bis 8 : 1, vorzugsweise 1 : 1 bis 6 : 1, und besonders bevorzugt 2 : 1 bis 3 : 1, beträgt.

**2.** Modifizierter Katalysator mit erhöhter Aktivität zur Herstellung von ortho-alkylierten Phenolen,
dadurch gekennzeichnet,
daß der modifizierte Katalysator
a) aus dem zu alkylierenden (2-Alkyl-)Phenol
b) einem Polyhalogenphenol als Cokatalysator
und
c) einer aluminiumorganischen Verbindung der allgemeinen Formel

$AlR_{(3-n)}X_n$,

in der n = 0 oder 1 ist, R für einen Alkylrest mit 1 bis 4 C-Atomen und X für Chlor oder Brom oder Wasserstoff steht,
gebildet wird und daß das molare Verhältnis zwischen den Katalysatorkomponenten b) und c) 0,5 bis 8 : 1 und vorzugsweise 2 : 1 bis 3 : 1 beträgt.

**3.** Katalysator nach Anspruch 2,
dadurch gekennzeichnet,
daß als aluminiumorganische Verbindung Aluminiumtriethyl verwandt wird.

**4.** Katalysator nach den Ansprüchen 2 bis 3,
dadurch gekennzeichnet,
daß als Cokatalysatoren Tri-, Tetra- und Pentahalogenphenole eingesetzt werden, bevorzugt 2,4,6-Trichlorphenol, Pentafluorphenol und besonders bevorzugt Pentachlorphenol.

**5.** 2-Cycloctyl-6-tert.butylphenol aus 2-Cyclooctylphenol und Isobuten, hergestellt gemäß Anspruch 1.

Europäisches Patentamt

# EUROPÄISCHER RECHERCHENBERICHT

Nummer der Anmeldung

EP 92 11 6506

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 438 641 (HÜLS AKTIENGESELLSCHAFT)<br>* das ganze Dokument * | 1,2 | C07C37/14<br>C07C39/06<br>C07C39/17 |
| D,A | US-A-3 355 504 (T.H. COFFIELD ET AL.)<br>* das ganze Dokument * | 1,2 | |
| D,A | US-A-3 426 082 (R.P. CURRY ET AL.)<br>* das ganze Dokument; insbesondere Spalte 4, Zeile 17 - Zeile 39 * | 1,2 | |

RECHERCHIERTE SACHGEBIETE (Int. Cl.5)

C07C

Der vorliegende Recherchenbericht wurde für alle Patentansprüche erstellt

| Recherchenort | Abschlußdatum der Recherche | Prüfer |
|---|---|---|
| DEN HAAG | 18 FEBRUAR 1993 | FINK D.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)